# EUROPEAN PATENT APPLICATION

(11) **EP 1 157 700 A1**
(43) Date of publication of application: **28.11.2001**
(21) Application number: 00906668.9
(22) Date of filing: 03.03.2000
(51) Int. Cl.: A61K 35/74, A61K 31/715, A61K 31/7088, A61P 31/18

(54) **ANTI-HIV AGENT**

(30) Priority: 09.03.1999 US 264686
(71) Applicant: Zeria Pharmaceutical Co., Ltd., Tokyo 103-8351 (JP); Maruyama, Natsu, Tokyo 113-0023 (JP)
(72) Inventor: SUZUKI, Fujio, Galveston, TX 77551 (US); SASAKI, Hidetaka, Galveston, TX 77551 (US); KOBAYASHI, Makiko, Galveston, TX 77550 (US)
(74) Representative: Hartz, Nikolai F., Dr.
(86) International application number: PCT/JP00/01276
(87) International publication number: WO 00/53203

(57) **Abstract**

The present invention relates to an anti-HIV agent which comprises as an effective ingredient a composition (a) containing as a primary component a polysaccharide obtained from a hot aqueous solvent extract of tubercle bacillus.

## Description

### Field of the Invention:

The present invention relates to an anti-HIV (human immunodeficiency virus) agent.

### Background of the Invention

AIDS is a disease caused by an HIV infection, and the number of patients suffering from this disease has markedly increased in recent years. The therapy for AIDS has involved use of nucleoside-type anti-HIV agents, such as Zidovudine (Azidothymidine, AZT) and Didanosine (ddI).

Unfortunately, these conventional anti-HIV agents do not provide a sufficient therapeutic effect. Consequently, development of a new anti-HIV agent which exhibits an enhanced, and thus adequate, therapeutic effect is required.

### Disclosure of the Invention

With the above in mind, the present inventors have conducted an intensive study to search for a new anti-HIV agent, thus coming to the discovery that a hot aqueous solvent extract of tubercle bacillus, by itself, exhibits an anti-HIV effect. As a result, the present invention was accomplished as hereinafter described in detail.

Namely, it is an object of the present invention to provide an anti-HIV agent which comprises as an effective ingredient a composition containing as a primary component a polysaccharide obtained from a hot aqueous solvent extract of tubercle bacillus.

It is also an object of the present invention to provide use of the above-mentioned composition for preparing an anti-HIV agent.

Yet another object of the present invention is to provide a method for inhibiting the replication of HIV, which comprises subjecting HIV-infected cells to the above-mentioned composition.

### BRIEF DESCRIPTION OF DRAWINGS

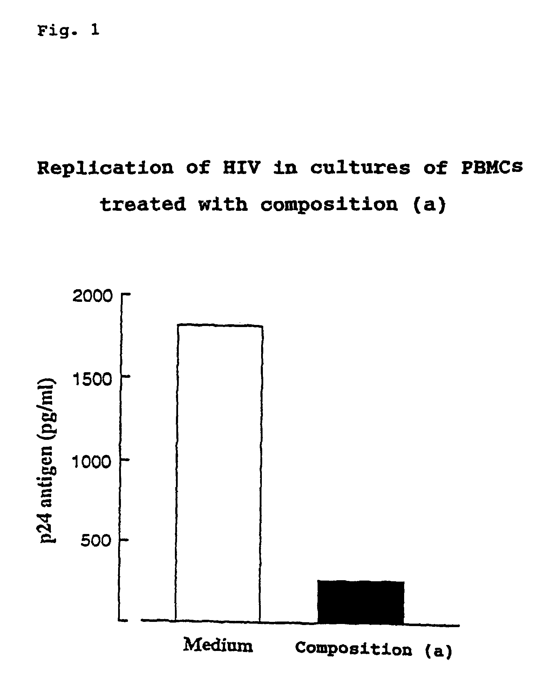

Fig. 1 illustrates the anti-HIV effect of the composition of the present invention on the replication of HIV in cultures.

### DETAILED DESCRIPTION OF THE PREFERRED EMBODIMENTS

The composition which is used in the present invention; i.e., a composition containing as its primary component a polysaccharide derived from a hot aqueous solvent extract of tubercle bacillus, has previously shown to recover the white blood cell count that was reduced by radiotherapy for cancers (see, among others, "The Clinical Report (Basic and Clinical Report)," 24(4), 1973(1990) and *"Nippon Igaku Hoshasen Gakkai Zasshi,"* 50(8), 993 (1990)).

However, the effect of the present composition, i.e., hot aqueous solvent extract of tubercle bacillus, on HIV was unknown prior to the present invention.

The present composition herein designated "(a)" contains a polysaccharide as its primary component. Preferably, composition (a) contains a polysaccharide whose primary constituents are arabinose, mannose and glucose, as well as a small amount of nucleic acid. Preferably, the polysaccharide has a molecular weight ranging from about 5 x 10² to 5 x 10⁴ as measured by the gel filtration method. The nucleic acid content of composition (a) is preferably about 0.05-0.3 wt.%. The composition (a) may contain about 1-5 wt.% the protein. Preferably, the mannose content of the polysaccharide is about 10-72 wt.%, the arabinose content about 3-30 wt.%, and the glucose content about 5-30 wt.%. The composition (a) of particular preference has the mannose content about 40-50 wt.%, the arabinose content about 15-25 wt.%, and the glucose content about 5-15 wt.%.

Composition (a) is obtained by purifying a hot-aqueous solvent extract of tubercle bacillus. More specifically, composition (a) is obtained by subjecting cells of tubercle bacillus to extraction with hot aqueous solvent. The extract is then subjected to protein removal treatment and to treatment for removing polysaccharides of molecular weight of about 10⁵ or more. The tubercle bacillus may be selected from both types of human *Mycobacteria* and non-human *Mycobacteria,* but human *Mycobacteria* are preferred. Examples of non-human *Mycobacteria* include *Mycobacterium bovis, Mycobacterium avium, Mycobacterium microti, Mycobacterium kansasii, Mycobacterium marium* and *Mycobacterium intracellulae.* Examples of human *Mycobacteria* include Aoyama B, H37Rv and H37Ra with Aoyama B being particularly preferred. The extraction with hot aqueous solvent is more accurately performed with aqueous solvent of a temperature of about 80°C to 120°C. Examples of aqueous solvent include fresh water, saline, sea water and sodium hydroxide solution with fresh water being preferred. The protein removal treatment is more thoroughly performed by causing proteins to precipitate by use of a protein precipitant such as sulfosalicyclic acid, trichloroacetic acid, or phosphotungstic acid, followed by the subsequent collection of the supernatant. The treatment to remove polysaccharides at a molecular weight of 10⁵ or more, is preferably performed by causing polysaccharides or high molecular weight to precipitate by use of ethanol, methanol, or acetone in a suitable amount, followed by the subsequent collection of the supernatant.

As shown in Examples, composition (a) has proved to be itself effective for inhibiting the replication of HIV (see Fig. 1).

In accordance with the present invention, composition (a) may be directly applied to HIV-infected cells so as to act on the cells. Composition (a) is preferably present in an amount of approximately 1-1000 µg/ml, with a more particular presence of 5-500 µg/ml, in the *in vitro* system. Also composition (a) is preferably administered by injection with a preference of a subcutaneous injection, in the *in vivo* case.

As calculated in terms of the saccharide content of arabinose in the *in vivo* case, the dose of composition (a) is preferably about 2-200 µg per day, more preferably about 20-100 µg per day.

When composition (a) is administered to a subject in need thereof, it is preferably prepared in the form of pharmaceutical compositions suitable for the aforementioned administration routes by incorporating thereto a generally employed, pharmaceutically acceptable carrier. Examples of carriers useful for the preparation of pharmaceutical compositions include; vehicles, binders, lubricants, disintegrants, coating agents, emulsifiers, suspensions, solvents, stabilizers, absorption aids, water for injection use, and tonicity agents.

### Examples

Reference to certain examples, which are provided solely for purposes of illustration and which are not intended to be limitative, will now be used to further describe the present invention.

### Referential example 1 (Preparation of Composition (a)):

*Mycobacterium tuberculosis* strain Aoyama B, which had been lyophilized and stored at -20°C, was subjected to seed culture at 37 ± 1°C in a Sauton-potato medium⁽¹⁾. The resultant cells were inoculated to a production medium⁽²⁾ and incubated for 5-7 weeks at 37 ± 1°C. Next, the harvested cells were washed with fresh water for injection use, followed by fresh water for injection use being added to the wet cells, at an amount 20 times the weight of the wet cells. To thereby obtain an extract, the mixture was heated to 100°C for 120 minutes. The extract was filtered by use of a 0.45 µm-membrane filter and concentrated under reduced pressure so that the saccharide content (converted to D-arabinose by the phenol-sulfuric acid method) fell within the range of 4.0-6.0 mg/ml, to thereby obtain a concentrate. Subsequently, in order to remove proteins, 1%(w/v) sulfosalicylic acid was added to the concentrate. The mixture was allowed to stand for 15-20 minutes at a temperature no higher than 10°C. Precipitates were removed by centrifugal separation (at a temperature of 10°C or lower, 1,150 x g, 10 minutes), to thereby recover the supernatant. The protein concentration of the supernatant was not more than 0.30 mg/ml (Lowry method, calculated as tyrosine). The supernatant was further processed to remove sulfosalicylic acid until the content of sulfosalicylic acid fell below the detection threshold (10 ppm or less, colorimetric method using ferric chloride solution). To have the saccharide content fall within the range of 1.8-2.2 mg/ml, the resultant solution was concentrated under reduced pressure. The concentrate was then combined with sodium chloride (0.9% (w/v)) and cold ethanol having a volume equal to that of the concentrate. The mixture was allowed to stand for no less than 40 hours at a temperature no higher than 10°C, then the precipitated polysaccharides of high molecular weight were removed by centrifugal separation (at a temperature of 10°C or lower, 2,040 x g, 10 minutes). Subsequently, the supernatant was combined with four times the volume of cold ethanol, and the mixture was allowed to stand for no less than 40 hours at a temperature no higher than 10°C, to recover precipitates by centrifugation (at a temperature of 10°C or lower, 2,040 x g, 10 minutes). The precipitates were dissolved in water for injection use, and after the saccharide content was adjusted to 1.8-2.2 mg/ml, the solution was subjected to filtration by use of a 0.45 µm membrane filter and to sterilization in an autoclave (121°C, 20 minutes), to thereby prepare a composition (a) solution.

### (1) Sauton-potato medium

Washed potato slices were soaked in a Sauton medium, followed by sterilization for 15 minutes at 115°C. The resultant material was used as a Sauton-potato medium.

| Sauton Medium | |
|---|---|
| L-asparagine (monohydrate) | 4.0 g |
| Citric acid (monohydrate) | 2.0 g |
| Magnesium sulfate (heptahydrate) | 0.5 g |
| Potassium monohydrogenphosphate (anhydrate) | 0.5 g |
| Ammonium iron citrate | 0.05 g |
| Glycerol | 60 ml |

The ingredients above were dissolved in water to make a total volume of 1,000 ml. By use of sodium hydroxide solution, the pH was adjusted to 7.0-7.3.

### (2) Production medium

| | |
|---|---|
| L-asparagine (monohydrate) | 4.0 g |
| Citric acid (monohydrate) | 2.0 g |
| Magnesium sulfate (heptahydrate) | 0.5 g |
| Potassium monohydrogenphosphate (anhydrate) | 0.5 g |
| Ammonium iron citrate | 0.05 g |
| Glycerol | 60 ml |

The ingredients above were dissolved in water to make a total volume of 1,000 ml, followed by sterilization in an autoclave (121°C, 20 minutes). By use of sodium hydroxide solution, the pH was adjusted to 7.0-7.3.

The physicochemical properties of the composition (a) solution were as follows.
(1) Appearance: Pale yellow clear liquid
(2) pH: 4.50-5.30
(3) Protein content: 3.5 wt.% (as an amino acid, in a freeze-dried product)
(4) Nucleic acid content: 0.1 wt.% (in a freeze-dried product)
(5) Constituent monosaccharides of polysaccharide:
   Mannose 43.4 wt.%,
   Arabinose 18.2 wt.%, and
   Glucose 10.4 wt.%.

Methods for determining the constituent monosaccharides of polysaccharide:
The polysaccharide was hydrolyzed with 2N trifluoroacetic acid for 2 hours at 100°C, and then subjected to liquid chromatography making use of 2-cyanoacetamide fluoroscein derivative (S. Honda, *et al*., Anal. Chem., 52, 1079 (1980)).

### Test Example 1:

HIV-1 IIIB (0.01 M.O.I.) was subjected to a two-hour absorption by peripheral blood mononuclear cells (PBMCs, 2 x 10⁶ cells/ml, derived from a seronegative donor) activated with phytohemagglutinin (PHA), to thereby prepare HIV-infected PBMCs. The HIV-infected PBMCs were cultured in the RPMI-1640 medium supplemented with 20% FCS and 20 U/ml of IL-2 at 37°C in the presence of composition (a). Seven days after the infection, HIV-1 p24 antigens present in the culture fluids were quantified by ELISA.

As is apparent from the results shown in Fig. 1, the addition of a 100 µg/ml dose of composition (a) reduces the amount of HIV-1 p24 antigens, proving that the composition provides an anti-HIV effect.

### Industrial Applicability of the Invention

As described above in detail, the present invention provides a significantly effective ability to inhibit replication of HIV in vitro and in vivo.

## Claims

1. An anti-HIV agent which comprises as an effective ingredient a composition (a) containing as a primary component a polysaccharide obtained from a hot aqueous solvent extract of tubercle bacillus.

2. The anti-HIV agent of Claim 1, wherein the composition (a) comprises a polysaccharide constituted by arabinose, mannose, and glucose as primary constituent monosaccharides, and nucleic acid.

3. The anti-HIV agent of Claim 1, wherein the composition (a) comprises a polysaccharide having a molecular weight of 5 x 10² - 5 x 10⁴ (as determined by gel filtration) and constituted by arabinose, mannose, and glucose as primary constituent monosaccharides, and nucleic acid.

4. The anti-HIV agent of Claim 1, wherein the tubercle bacillus is a human tubercle bacillus.

5. The anti-HIV agent of Claim 4, wherein the human tubercle bacillus is an Aoyama B strain.

6. Use of the composition (a) described in any one of Claims 1 to 5 for preparing an anti-HIV agent.

7. A method for inhibiting the replication of HIV, which comprises subjecting HIV-infected cells to a composition (a) comprising as a primary component a polysaccharide obtained from a hot aqueous solvent extract of tubercle bacillus.

8. The method of Claim 7, wherein the composition (a) comprises a polysaccharide constituted by arabinose, mannose, and glucose as primary constituent monosaccharides, and nucleic acid.

9. The method of Claim 7, wherein the composition (a) comprises a polysaccharide having a molecular weight of 5 x 10² - 5 x 10⁴ (as determined by gel filtration) and constituted by arabinose, mannose, and glucose as primary constituent monosaccharides, and nucleic acid.

10. The method of Claim 7, wherein the tubercle bacillus is a human tubercle bacillus.

11. The method of Claim 10, wherein the human tubercle bacillus is an Aoyama B strain..
